# EUROPEAN PATENT APPLICATION

(11) **EP 2 926 816 A1**
(43) Date of publication of application: **07.10.2015**
(21) Application number: 13860352.7
(22) Date of filing: 11.11.2013
(51) Int. Cl.: A61K 31/57, A61K 31/55, A61K 31/445, A61K 31/4545, A61K 31/4465, A61K 9/08, A61P 11/00, A61P 27/14, A61P 37/08

(54) **PHARMACEUTICAL COMPOSITION COMPRISING DESLORATADINE AND PREDNISOLONE AND USE THEREOF**

(30) Priority: 03.12.2012 BR 102012030828
(71) Applicant: EMS S.A., 13186-901 Sao Paulo - SP (BR)
(72) Inventor: AMARAL, Erich Bertoldi, 13186-901 Hortolândia - SP (BR); COVESI, Leticia Khater, 13186-901 Hortolândia - SP (BR); REGO, Pedro Bordeaux, 13186-901 Hortolândia - SP (BR); ANDRADE, Pedro Henrique Lage de, 13186-901 Hortolândia - SP (BR); SAKASAQUI, Renato Kendy, 13186-901 Hortolândia - SP (BR)
(74) Representative: Cabinet Plasseraud
(86) International application number: PCT/BR2013/000484
(87) International publication number: WO 2014/085884

(57) **Abstract**

The present invention relates to a liquid pharmaceutical composition for oral administration and to the use thereof, said composition being characterized by comprising: (i) a therapeutically effective quantity of desloratadine or a pharmaceutically acceptable salt thereof; (ii) a therapeutically effective quantity of prednisolone or a pharmaceutically acceptable salt thereof; (iii) a selection of pharmaceutically acceptable excipients wherein the flavoring excipient used is free of vanillin and derivative thereof, and wherein the pH of said composition is maintained at a value of between 6.0 and 7.5.

In the preferred embodiment, the composition of the invention is an antihistamine composition for oral administration, in the form of sugar-free syrups, intended for pediatric use.

## Description

### FIELD OF THE INVENTION

The present invention relates to a pharmaceutical formulation containing the active ingredients desloratadine and prednisolone, or one of their pharmaceutically acceptable salts, having improved stability and being preferably suitable for pediatric use, or for people with difficulty administering non-liquid oral forms. The pharmaceutical formulation of the invention is improved compared to known liquid formulations of desloratadine, particularly in relation to the active ingredients which should have enhanced stability, and the non-active ingredients which should have minimized toxicity and improved organoleptic properties.

### BACKGROUND

Histamine is the main mediator in the development of allergic rhinitis, in both initial and late phase reactions. In the initial phase, the pre-sensitized mast cells exposed to allergen release histamine to cause acute symptoms. In the late phase, inflammatory mediators lead to the activation of eosinophils and basophils, resulting in additional histamine release and inflammation (see Shao-Hua Xie Heng and He. Roles of histamine and its receptors in allergic and inflammatory bowel diseases." World J. Gastroenterol 2005, 11(19): 2851-2857; Bloemen, K. and collaborators. "The allergic cascade: Review of the most important molecules in the asthmatic lung." Immunology Letters, 113, 2007, 6-18). Symptoms directly attributable to the late stage release of histamine include sneezing, itching, runny nose and nasal congestion. The relief of symptoms of allergic rhinitis can be obtained by blocking the action of histamine, which is the main target of allergic conditions therapy, and for the treatment of inflammation that has developed (see US6709676 and US7618649).

In the same way that intranasal antihistamines are recommended, oral antihistamines are also recommended as first-line therapy in the treatment of allergic rhinitis. In general, the second-generation drugs are preferred because they have the advantage of not causing sedation. Currently, the second generation drugs available on the market are those based on loratadine, desloratadine, fexofenadine, cetirizine, levocetirizine and acrivastine, the latter only in combination with pseudoephedrine.

Unlike intranasal antihistamines that target nasal symptoms, oral antihistamines are intended primarily for the symptoms associated with histamine, such as crisis sneezing, runny nose, itching, watery and red eyes. Oral antihistamines have some effect on nasal congestion but to a lesser extent when compared to intranasal antihistamines (see Scarupa, M.D. and Kaliner, M.A. "In-depth Review of Allergic Rhinitis." WAO (World Allergy Organization), available on the Web in June 2005 (http://www.worldallergy.org/professional/allergic diseases center/rhinitis/r hinitis indepth.php) ; Kim, H. "Allergic Rhinitis." Allergy / Asthma Information Association. Available on the Web at http://www.aaia.ca/en/allergic rhinitis.htm, accessed 10/30/2012; Lehman J.M. and Blaiss, M.S. "Selecting the Optimal Oral antihistamine for Patients with Allergic Rhinitis." Drugs 2006, 66 (18): 2309-2319).

In a study conducted with 331 patients, the effects of administration of a daily dose of 5 mg desloratadine was compared with treatment with placebo, so that desloratadine was effective in reducing overall symptoms, the symptoms of asthma and the symptoms of nasal congestion (see Berger W.E., Schenkel E.J., and Mansfield L.E. Desloratadine Study Group. Safety and efficacy of desloratadine 5 mg in asthma patients with seasonal allergic rhinitis and nasal congestion. Ann Allergy Asthma Immunol 89: 485- 491, 2002).

Among the dosage forms for oral administration suitable for pediatric use or for people suffering from limited intake of solid forms, the aqueous solutions or dispersions may be cited, particularly syrups and sugar-free syrups (solutions without added sugar or sugar-free). Syrups are defined as an aqueous pharmaceutical composition characterized by having high viscosity presenting less than 45% sucrose or other sugars, and which contain flavoring agents. The sugar-free syrups are pharmaceutical forms, for oral use, sweetened and sugar-free, and can be in the form of pharmaceutical solutions or dispersions; they are also known as "sugar-free syrups or syrups for diabetics" and are used to replace the classic form of syrup vehicle; whose formulation is comprised of aqueous carriers, or sorbitol-based, and even as a mixture of glycerin and water, and may also be found in the form of a colloidal dispersion sweetened for internal use. However, despite their preference from the point of view of ease in administration, liquid dosage forms are more likely to present stability problems of active ingredients, since oxidation reactions are enhanced in an aqueous medium. In addition, another challenge faced in the development of liquid dosage forms is to obtain acceptable organoleptic properties, since the normally unpleasant taste of some drugs, such as desloratadine, is always more pronounced when they are present in solution than when incorporated into solid forms. However, the solutions can become more palatable by adding non-active flavoring components and sweetening agents which provide a greater chance of patient compliance, especially in the treatment of children. (see Billany, M. solutions. In: Aulton, M.E. Design of Pharmaceutical Forms. 2nd ed. Porto Alegre: Artmed, 2005. chapter. 21, p.318-319).

The main events related to the stability of active ingredients in such dosage forms include the tendency to precipitation and formation of degradation products from the contact or exposure of the active ingredients with non-active ingredients. Desloratadine is a representative example of such stabilization problems of active ingredients in a liquid medium. These problems are accentuated in the presence of a second active ingredient that can interact with desloratadine, resulting in a precipitation event, or even a change of color.

In addition, the fact is also known that corticosteroid salts exhibit stability problems when these compounds are incorporated into aqueous compositions. The degradation of the steroid can be visualized by precipitate formation and the development of a yellow color, as mentioned in document US3898330.

Various formulations have been proposed to reduce the instability of desloratadine, alone or in combination with other principle active ingredients, both in terms of precipitate formation and degradation products. For example, in document WO2005115353, an oral aqueous liquid pharmaceutical composition comprising a suspension system based on xanthan gum and microcrystalline cellulose / sodium carboxymethyl cellulose and at least one active ingredient that is substantially insoluble (preferably ibuprofen) in water, or is designed to be soluble in the aqueous composition is described, as desloratadine. In a further embodiment, said composition contains a second soluble principle active ingredient, for example, a decongestant.

Document WO0051605 describes compositions and methods of treatment of atopic dermatitis, angioederma and other disorders using antihistamines and glucocorticoids, preferably betamethasone and loratadine, and also mentions desloratadine and prednisolone as examples of principle active ingredients belonging to these classes of drugs. Although a combination of a non-sedating antihistamine with a glucocorticoid is mentioned herein, no mention is made of stabilizing the active ingredients present in a liquid dosage form for oral administration. Document WO2005027839 also describes the combination of an antihistamine, for example, desloratadine, with one or more additional therapeutic agents, for example, a steroid such as prednisolone for the treatment of immunoinflammatory disorders. Also, in WO2005027839, no known solution is disclosed for the instability of any of the active ingredients, as for example, desloratadine, nor is any liquid pharmaceutical form for oral administration exemplified.

Document US2012022094 describes stable pharmaceutical formulations of desloratadine, in syrup form, whose formulations may contain one or more additional principle active ingredients selected from decongestants, expectorants, non-steroidal anti-inflammatory drugs, among others. According to this document, the stability of desloratadine is achieved by means of maintaining the pH at a value above 4.5.

Document US6514520 describes an antihistaminic formulation in the form of syrup, comprising desloratadine and a specified amount of an aminopolycarboxylic acid or a salt thereof, e.g., EDTA, said formulation further containing a decongestant (preferably pseudoephedrine or phenylpropanolamine), analgesic, antitussive, or an expectorant.

Documents WO2006069213 and WO2008005267 describe desloratadine formulations in the form of syrup, whose stability is also attributed to the maintenance of pH at a value above 4.5, preferably between 4.5 and 6.5, more preferably between 5 and 6 and even more preferably 5.5. It is also mentioned in these documents that the formulation can comprise one or more additional therapeutic agents, being mentioned, among others, steroidal compounds such as prednisone and prednisolone. However, no example is presented of the association of desloratadine with another therapeutic agent. In other words, there is no evidence that adjusting the formulation pH to the range 4.5 and 6.5 is sufficient to stabilize a formulation containing desloratadine and prednisolone.

There are several difficulties in formulating liquid compositions, for example, oral solutions and syrups, containing desloratadine, which can often be detected by a color change of the formulation. For example, document US6514520 mentions that a syrup, available on the market, containing loratadine and citric acid, artificial flavoring, glycerin, propylene glycol, sodium benzoate, sucrose and water, having a pH value in the range 2-4, under certain storage conditions, in contact with air, results in the loss of loratadine and the concomitant generation of impurities. The same phenomenon occurs with desloratadine (see column 1, lines s 54-63). Certain common excipients such as lactose, magnesium stearate and microcrystalline stearate have also been reported to be incompatible with desloratadine, resulting in degradation of the desloratadine detectable by the appearance of pink color (see the third paragraph on page 4 WO2006069213). Additionally, document WO2008005267 mentions that the antihistamine syrup formulation disclosed in patent US6414520, comprising desloratadine and about 0.05 to about 5 mg / ml of an aminopolycarboxylic acid or a salt thereof, results in a bright pink color when stored in the absence of light, which requires the addition of dye to mask this degradation of desloratadine (see page 2, second paragraph of WO2008005267).

Benzoates, as additives (preservatives) of food and medicine, are related to various disorders and diseases. Clinical data indicate that this preservative can produce non immunologic contact urticaria. Other reported adverse effects include anaphylaxis and urticarial reactions (see Rowe, R.C., Sheskey P.J.; Owen, S.C. Handbook of Pharmaceutical Excipients. Pharmaceutical Press, Fifth Edition, p.663, 2006). Recent studies have linked certain artificial colorants and benzoate with the behavior of hyperactive children and atopy (see, e.g., Bateman, B. et al. "The effects of a double blind, placebo controlled, artificial food colorings and benzoate preservative challenge, on hyperactivity in a general population sample of preschool children. Arch Dis Child 2005, 90 (8) : 875, available on the Web at http://www.ncbi.nlm.nih.gov/pubmed/15155391; "Artificial food coloring and hyperactivity Symptoms in children. "Prescrire Int. October / 2009 18 (103) : 215, available on the Web at http://www.ncbi.nlm.nih.gov/pubmed/19882794). Benzoates can also initiate allergies such as rash and asthma and have been linked to causes of brain damage (see Pandey, R.M. and Updhyay, S.K. "Food Additive "page 11, item 4, 2012, available on the Web at http://www.intechopen.com/books/food-additive/foodadditive). When present in liquid mixtures (e.g., soft drinks) containing ascorbic acid or citric acid, benzoates can be reacted in an acidic medium with the formation of benzene, a known carcinogen (see, for example, Morsi, RMY and collaborators. "Probability of benzene forming in Egyptian Non-Alcohol Carbonated Soft Drinks." Australian Journal of Basic and Applied Sciences, 6 (3): 271-278, 2012) which may cause cancer, for example leukemia. In addition to all the effects described above, it is also known that sodium benzoate preservative has a reduced activity in the presence of nonionic surfactants such as polysorbate, for example (see Rowe, R.C., Sheskey PJ; Owen, S.C. Handbook of Pharmaceutical Excipients. Pharmaceutical Press, Fifth Edition, p.663, 2006).

Therefore, the therapy for allergic rhinitis and other allergic disorders treatable with systemic antihistamines still lacks a stable product in terms of shelf life, which in addition to antihistaminic activity also allows the treatment of inflammation resulting from allergic reactions to substances of the class of corticosteroids. Thus, the provision of oral liquid formulations is urgent, especially for pediatric use, containing a second generation antihistamine, e.g., desloratadine, and a corticosteroid, e.g., prednisolone, which avoids the addition of excipients, e.g., dyes, preservatives, sugars or sweeteners, that can react with one or two active ingredients, but are provided with suitable organoleptic properties to ensure adherence to the individual to treatment.

### SUMMARY OF THE INVENTION

The present invention aims to provide a stable pharmaceutical composition in oral solution or sugar-free syrup based on a combination of desloratadine and prednisolone or a pharmaceutically acceptable salt thereof, in which physicochemical characteristics such as color, flavor and solubility characteristics remain unchanged for long periods. Additionally, from the therapeutic point of view, the combination of the invention reduces the adverse effects of desloratadine.

The invention is embodied in the form of an oral liquid pharmaceutical composition comprising (i) a therapeutically effective amount of desloratadine or a pharmaceutically acceptable salt thereof; (ii) a therapeutically effective amount of prednisolone or a pharmaceutically acceptable salt thereof and (iii) a pharmaceutically acceptable excipient together wherein the excipient is free of the flavoring agent vanillin or a derivative thereof, and wherein the pH of said composition is maintained at a value in the range between 6.0 and 7.5.

A second aspect of the present invention is the oral liquid pharmaceutical composition comprising (i) a therapeutically effective amount of desloratadine or a pharmaceutically acceptable salt thereof; (ii) a therapeutically effective amount of prednisolone or a pharmaceutically acceptable salt thereof and (iii) a group of pharmaceutically acceptable excipients containing polysorbate 20 as a surfactant component suitable for maintaining compatibility of both desloratadine and prednisolone in solution, with the pH of said composition in the range between 6.0 and 7.5.

A third aspect of the invention relates to an oral liquid pharmaceutical composition, particularly for pediatric use, containing the principle active ingredients desloratadine and prednisolone or a pharmaceutically acceptable salt thereof, together with a pharmaceutically acceptable excipient, said composition being absent of sugars, colorants and preservatives which are harmful to health, such as sodium benzoate.

A fourth aspect of the invention relates to the use of the pharmaceutical composition of the invention for the preparation of a medication for the treatment of children suffering from allergic disorders or inflammation resulting from said allergic manifestations.

### DETAILED DESCRIPTION OF THE INVENTION

The present invention relates to a pharmaceutical formulation for oral administration, directed to the treatment of allergic manifestations or disorders, which combines the therapy of allergic conditions with immune inflammatory response. Specifically, the invention relates to the combination of an active ingredient practically insoluble in water (desloratadine) with another active ingredient highly soluble in water, present in a liquid composition which ensures the stability of these active ingredients with regard to the precipitation of compounds and the formation of degradation products.

Desloratadine, a substance poorly soluble in water, moderately soluble in propylene glycol and very soluble in ethyl alcohol, is the main principal active ingredient metabolite of loratadine and has pharmacodynamic activity qualitatively similar to that of loratadine, but with a potency of about 10 to about 20 times greater compared to the latter. It is an H1 receptor antagonist, which qualifies as effective in relief of symptoms associated with seasonal allergic rhinitis and some other allergic reactions. Histamine H1 receptor antagonists are important as a first line of drugs for symptomatic treatment of allergic manifestations. One of the advantageous features of desloratadine in relation to other antihistamines is that is has no sedative effect.

Prednisolone is a synthetic adrenocorticosteroid analogue, being a steroid in the form of a free or esterified alcohol, with predominant glucocorticoid properties. This synthetic compound can reproduce some effects of endogenous glucocorticoids, but after administration of high doses may cause effects that do not necessarily resemble those of adrenocortical hormones.

Prednisolone has multiple actions that provide anti-inflammatory effects that result in their use in the treatment of several pathologies such as asthma. Among other pharmacological properties, it reduces inflammation by stabilizing lysosomes of neutrophils and by inducing the synthesis of anti-inflammatory proteins known as lipocortins that inhibit phospholipase A2, and inhibit the synthesis of prostaglandins and lipoxygenase products. Prednisolone may also cause some metabolic effects based on its glucocorticoid properties. These include: promoting gluconeogenesis, increased deposition of glycogen in the liver, inhibition of glucose utilization, anti-insulin activity, increased protein catabolism, increased lipolysis, stimulating the synthesis and storage of fat, increased glomerular filtration rate and resulting increase in urinary excretion of urate and increased calcium excretion.

Desloratadine and prednisolone sodium phosphate have markedly different physical and chemical characteristics. The desloratadine base is not protonated, with low solubility in water, presenting two pKa values between 4.41 and 9.97 (see Popovic, G.; Cakar, M. Agbaba, D. Acid-base equilibria and solubility of desloratadine and loratadine in water and micellar medium. Journal of Pharmaceutical and Biomedical Analysis, V.49, p.42-47, 2009). The sodium form of prednisolone via a phosphate salt type makes this molecule extremely water soluble. Given these differences, it was necessary to establish a pharmacotechnical system, in the present invention, capable of supporting both solubilized and stabilized chemical species.

As described by Popovic et al (2009), the solubility of poorly soluble drugs, such as desloratadine, can be improved by modifying the acid-base properties of protholiths through, for example, the use of surfactants to assist in the formation of micellar surfactant mediums. The use of polysorbate in the present invention meets this theory, since it provides the formation of a solubilizing system which maintains both the prednisolone sodium phosphate as well as the desloratadine dissolved in the same medium. Additionally, the invention also features EDTA as a sequestering agent, since it has the property of complexing trace metals present in excipients such as buffer salts; those salts that can lead to oxidation of chemical species such as prednisolone (see Oesterling, T.O.; Guttman D.E. Factors influencing stability of Prednisolone in aqueous solution. Journal of Pharmaceutical Sciences, V.53, p.1189-1192, 1964). According to the same reference, working in an ideal pH range is essential for maintaining the stability of the active ingredient in a liquid dosage form.

In view of the above chemical prerogatives, the invention is embodied in the form of an oral liquid pharmaceutical composition comprising (i) a therapeutically effective amount of desloratadine or a pharmaceutically acceptable salt thereof; (ii) a therapeutically effective amount of prednisolone or a pharmaceutically acceptable salt thereof, and (iii) a set of pharmaceutically and appropriate acceptable excipients to maintain stabilized in a solution of desloratadine and prednisolone, including a sequestering agent, so that the pH of said composition should be in the range between 6.0 and 7.5, by employing a suitable buffer system.

Sweetening agents, with sweetening properties, for use in the present invention include sucralose, sodium saccharin, potassium acesulfame, sorbitol, xylitol, maltodextrin, fructose, stevia, aspartame, sodium cyclamate, monoammonium glycyrrhizinate, and other selected sweeteners. Preferably, employed in the composition of the invention, is a set of sweeteners, saccharin, potassium acesulfame and sucralose, which complement their individual effects. Sweetening agents can be present, for example, in concentrations of 0.01% to 5.0%, preferably between 0.01 and 2.0%.

Suitable buffer systems employed in the present invention may include citric, phosphoric, tartaric, fumaric, maleic, and acetic acids and salts. The buffer system based on citrate typically consists of sodium citrate and citric acid whose proportion may lie in the range between 8:1 and 20:1, preferably in the range 17:1. A suitable buffer system for this type of association in the form of sugar-free syrup has a pH greater than 6.0; preferably in the range between 6.5 and 7, 5.

Usually, pharmaceutically acceptable solvents or carrier systems include water, alcohols and glycols, primarily propylene glycol, sorbitol, polyethylene glycol and / or glycerin. The use of alcohol in pediatric formulations is not recommended, due to the characteristic of toxicological risks of this solvent. The use of the combination of at least one of water, propylene glycol, sorbitol and glycerin is preferred. Preferably, the amount of sorbitol employed as a vehicle, should be between 20 and 50%, preferably between 25 and 35%. The amount of propylene glycol, employed to solubilize the essence and also the desloratadine, must be in a range between 10 to 30%, preferably between 14 and 20%.

Thickening agents for use in the present invention include hyetellose, guar gum, gelatin, xanthan gum, tamarind gum, tragacanth gum, karaya gum, water-soluble carboxyvinyl polymers (e.g., povidone), sodium carboxymethylcellulose, sodium alginate, pectin, carrageenan, polyethylene glycol, modified starch, cellulose derivatives such as carboxymethylcellulose, hydroxymethylcellulose, hydroxypropylmethylcellulose, hydroxyethylcellulose, methylcellulose and microcrystalline cellulose. Hyetellose is preferably used as a thickener of choice in a concentration of 0.1 to 2.0%, preferably between 0.1 and 0.5%.

In the preparation of liquid pharmaceutical formulations, several problems can arise. For example, formulations containing high concentrations of solvents have intrinsic antimicrobial activity. Propylene glycol (used as a vehicle and co-solvent), for example, has inherent antimicrobial activity. The use of essential oil based essences (organic material) increase the risk of contamination in a fluid system, and the use of at least one pharmaceutically acceptable preservative agent in the formulation is recommended. Thus, the addition of preservatives that are employed in oral solutions such as benzoic acid (from 0.1 to 0.15%), benzyl alcohol (up to 2.0%) and various combinations of methyl-, ethyl-, propyl-, and butylparabens (totaling a maximum of 0.2% paraben mixture) is commonly made. The preservative, according to the invention, is present in the composition in a concentration ranging from 0.05% to 2%. According to a preferred embodiment of the invention, the preservative is methylparaben, and is present in a concentration in the range of 0.05-0.5%, and is considered an effective preservative. More preferably, the preservative methylparaben is present at a concentration of 0.18%.

As already explained in the foundations of the invention, the presence of drugs with different solubility properties makes it necessary to use at least one surfactant in the formulation to provide the formation of a micellar system with solubilizing properties. In this sense, the present invention includes natural surfactants such as soybean lecithin, castor oil, corn oil, cottonseed oil, sunflower oil, peanut oil, and synthetics, such as polysorbate 20, 60 or 80 and mixtures thereof. Polysorbate 20 at a concentration in the range of 0.1 to 0.5%, presents an advantage over others since it has no residual flavoring property and therefore does not confer the sugar-free syrup with unpleasant organoleptic characteristics; for this reason it is the most preferred for use in the composition of the invention.

The incorporation of stabilizers in the formulation is also important in an aqueous medium, which is conducive to the occurrence of oxidation reactions, for example. Since prerequisites are part of an oral solution, the stabilizer should have high solubility in water and be safe for ingestion. Such features are covered by ethylenediaminetetraacetic acid, better known as EDTA, an organic compound that acts by complexation of free metals in solution (potential oxidizing agents), which is why it is also treated as a sequestering agent for metals. When employed in a formulation, EDTA or other chelating agents (edetate dipotassium, edetate disodium, sodium edetate, trisodium edetate) can be present in the range between 0.005 and 0.1% and can reach up to 5.0% depending on the formulation.

In order to mask the unpleasant taste of the drugs desloratadine and prednisolone sodium phosphate, the present invention includes some examples of selectable flavoring to improve the organoleptic properties of oral solutions, especially those with pediatric indication. Among them stand out some derivatives of essential oils such as mint, lemon, orange, peppermint, eucalyptus, and others based on organic synthetic blends like cherry, strawberry, pineapple, caramel, Tutti-Frutti, honey, fruit salad and a number of other essences that may be part of the scope of the invention. It has now also been verified that the components of vanillin or its derivatives, such as ethyl vanillin, present in essences such as tutti-frutti, must be removed from the mixture that composes the "bouquet" of the scent, given that vanillin or its derivatives react with desloratadine and / or prednisolone, causing the appearance of degradation products, evidenced by the development of pink color. Thus, according to the invention, the essence of tutti-frutti is preferably employed as long as the derivatives of its vanillin components are removed. Preferably, the essence of the invention is employed in a concentration in the range from 0.05 to 0.5%.

Various prior art patent documents mention the occurrence of the pinkish appearance of formulations containing desloratadine, propylene glycol and flavors; see for example, US6514520. In this case, the solution proposed in these documents was the addition of dye required to mask the appearance change. However, certain dyes can be allergenic and are not ideal to make up a formulation having therapeutic allergenic properties. Thus, the present invention provides stable dye-free formulations, which do not exhibit coloration with the passage of time. It has been found during the development of the formulation of the present invention, that in the presence of vanillin or vanillin derivatives (e.g., ethyl vanillin, methyl vanillin, etc.) in pharmaceutical compositions containing desloratadine or desloratadine and prednisolone, colorimetric reactions occur with desloratadine by changing, consequently, the color of the solution due to the degradation of desloratadine. Thus, the present invention provides a solution to avoid the degradation of desloratadine and the use of dyes in oral solutions such as syrups with pediatric indications which identify vanillin (or a derivative thereof) as the source of these problems, and thus proposes the elimination of this component of the essence to be employed in the formulation.

The present invention, in the most preferred embodiment, has an anti-histamine formulation in liquid sugar-free syrup form consisting of an antihistamine agent, preferably desloratadine, in combination with another chemical entity, with corticosteroid properties such as prednisolone sodium phosphate, in the presence of EDTA, sweeteners such as acesulfame potassium, sucralose and saccharin; a buffer system consisting of citric acid and sodium citrate dihydrate in proper proportion to maintain the pH of the formulation to a value in the range from 6.0 to 7.5; hyetellose as a thickener; methylparaben in the function of preservative; polysorbate 20 as a surfactant; propylene glycol and sorbitol as a solvent system; and an essence compatible with the active ingredients, preferably Tutti-Frutti, for masking the unpleasant taste characteristic of the same in the formulation, provided that said substance is exempt from the component of vanillin or a derivative thereof. It should be added that in the preferred embodiment of the invention, dyes and sodium benzoate and other allergenic and / or carcinogenic substances that may compromise the therapeutic effects of the formulation are absent.

Also in the preferred embodiment, the liquid composition of the invention is in the form of sugar-free syrup, and the pH value should be above 6.0. Preferably, the pH range should be between 6.0 and 7.5. More preferably a value of 6.5, and even more preferably a value amounting to 7.0.

Preferably, the amount of sorbitol employed as a carrier and sweetener should be between 20 and 50%, preferably between 25 and 35%.

The amount of propylene glycol, employed to facilitate solubilization of the essence of desloratadine, should be in a range between 10 to 30%, preferably between 14 and 20%.

The following are specific embodiments of the invention. However, it should be understood that such examples are provided for illustrative purposes only, and that various modifications or changes, in light of the embodiments disclosed herein, will be suggestive to specialists in the art and must be included within the spirit and scope of this disclosure and the scope of the accompanying claims.

### EXAMPLES

### Preparation of the Formulation of the Invention

The sugar-free syrup manipulation process is based on two concomitant steps: preparation of the prednisolone sodium phosphate phase in an aqueous medium comprising water and sorbitol, as well as the sweeteners, stabilizer, buffer system ingredients and preservative(s).

The desloratadine phase is prepared in parallel by solubilizing it in propylene glycol or glycerin, in the presence of the surfactant and essence. The last step is the addition of the desloratadine phase (oil) to the prednisolone sodium phosphate phase (aqueous).

The preparation procedure follows the following protocol:
1. In a suitable reactor, heat a volume of purified water of 400.000 L to a temperature in the range of 50-90° C. Subsequently add methylparaben under agitation, until complete solubilization.
2. Subsequently add the citric acid monohydrate and sodium citrate dihydrate under stirring until complete solubilization.
3. Adjust system temperature in the range of 50-80° C. Add the following raw materials under constant stirring: EDTA, acesulfame K, sucralose and saccharin. Mix until completely dissolved.
4. Add the hyetellose, under constant stirring, homogenizing until complete solubilization. Add prednisolone sodium phosphate, under stirring until complete solubilization.
5. Later add sorbitol and maintain under agitation.
6. In an auxiliary reactor, solubilize the desloratadine in propylene glycol.
7. In a stainless pot with sufficient capacity to homogenize the flavor tutti-frutti free of vanillin (or a derivative thereof) in polysorbate 20 and propylene glycol.
8. Pour the mixture prepared in item 8 over the mixture prepared in item 7 and mix.
9. Promote the transfer of the auxiliary reactor contents to the main reactor, under constant agitation.
10. Make up the volume to 1,000.000 L with purified water.
11. Check appearance and pH. Later filling in amber bottles according to specifications.

### Analytical Methodology Used

**Equipment and Materials:** HPLC ultrasound column: Symmetry C18 (4.6 x 150 mm), monobasic ammonium phosphate, hexylamine, acetonitrile, phosphoric acid, desloratadine, prednisolone sodium phosphate.

**Mobile phase:** Add 5.32g monobasic ammonium phosphate to a 4000 ml beaker and add 6.6g of cyclohexylamine to the same beaker and allow standing for 10 minutes. After resting, add 1850 mL of milli-Q water and stir until complete dissolution and adjust the pH value to 4.7 with phosphoric acid and mix with 460 mL of acetonitrile. Homogenize, filter and degasify.

**Preparation of Standard Stock Solution of Desloratadine:** Add 10 mg of desloratadine to a 100 mL volumetric flask, add 50 ml of diluent 1 (Phase Mobile - Ammonium phosphate buffer pH 4.7: acetonitrile 80:20) carry to ultrasound until completely solubilized and complete the volume with diluent 1. Final concentration: 0.1 mg of desloratadine / ml solution.

**Preparation of Standard Stock solution of Prednisolone Sodium Phosphate:** Add 22 mg of prednisolone sodium phosphate to a 10 mL volumetric flask, add 5ml of diluent 1, carry to ultrasound to completely solubilize and dilute with diluent 1. Final concentration: 2.2 mg of prednisolone sodium phosphate / ml solution.

**Standard preparation of Desloratadine + Prednisolone Sodium Phosphate:** Transfer an aliquot of 2.0 mL of Standard Stock Solution of desloratadine to a 10 mL volumetric flask, and into the same flask, transfer a 1.0 mL aliquot of Standard Stock Prednisolone Sodium Phosphate and complete with diluent 1. Mix and filter in a membrane of 0.45 of µm.
- Final concentration: 0.02 mg desloratadine / ml solution.
- Final concentration: 0.22 mg of prednisolone sodium phosphate / ml solution.
- Final concentration: 0.16 mg of prednisolone / ml solution.

**Sample solution:** Determine the density of the sample to be analyzed. Transfer the mass equivalent to 1 ml of the product to a 25 mL volumetric flask. Add 10 ml of diluent 1 and leave in ultrasound for 10 minutes waiting for the flask to cool and complete with diluent 1. Filter in 0.45 µm membrane and inject 20 µL of standard and sample solutions in HPLC.
- *Final concentration:* 0.02 *mg desloratadine* / *ml solution.*
- *Final concentration: 0.16 mg of prednisolone* / *ml solution.*

### Primary Packaging

Each bottle used as primary packaging consists of: A 120 ml Pet bottle with 24mm amber plastic cover and 24mm Pilfer seal.

### Example 1

A formulation was prepared according to the methodology of "Preparation of the Formulation of the Invention" described above, which, according to a preferred embodiment of the invention has the following composition:

| Ingredient | Concentration (mg/mL) |
|---|---|
| Desloratadine | 0.5 |
| Prednisolone sodium phosphate | 5.36* |
| Propylene glycol | 160.0 |
| Sorbitol | 300.0 |
| Disodium dihydrate edta | 1.0 |
| Sodium citrate dihydrate | 6.8 |
| Anhydrous citric acid | 0.395 |
| Sodium Saccarin | 1.0 |
| Sucralose | 2.0 |
| Acesulfame potassium | 0.8 |
| Methylparaben | 1.8 |
| Hyetellose | 1.25 |
| Polysorbate 20 | 2.0 |
| Tutti-Frutti aroma without vanillin component (or derivative of the same) | 1.0 |
| Purified Water | q.s.1.0 mL |

| | |
|---|---|
| *Equivalent to 4.00g of Prednisolone in base form. | |

The solution obtained in the form of sugar-free syrup for oral administration was packaged as described above and subjected to accelerated stability testing and validity for 24 months. The results are provided in Examples 2 and 3 below.

### EXAMPLE 2: Accelerated Stability Test of the Formulation of the Invention

The formulation of the invention, obtained according to the procedure of Example 1 was subjected to an accelerated stability study under the following conditions: Temperature: 40 ± 2° C; Relative humidity: 75 ± 5%. The results are shown in Table 1.

**Table 1: Accelerated Stability Study of the Formulation of the Invention Obtained in Example 1**

| Accelerated Stability (40 +/- 2°C / 75 +/- 5% RH) | | | | |
|---|---|---|---|---|
| Product: desloratadine + prednisolone sodium phosphate | | | Lot: PD06 | |
| Active Ingredients: desioratadine + prednisolone sodium phosphate | | | | |
| Manufacturer of the Active Ingredients: Cadila Healthare Limited/Henan Lihua | | | Lot of the Active Ingredients: M255891 / M241709 | |
| Packaging Materials Ambar glass flask | | | Dosage: 0.50 mg/mL + 2.00 mg/mL | |
| Pharmaceutical Form: Oral solution | | | | |
| Amount of samples analyzed per period: 03 units | | | Size of the lot: 5,00 Liters | |
| Fabrication: 08/2011 | | | Validity: 24 months | |
| Beginning of the study: 08/03/2011 | | | End of the study: 02/13/2012 | |

| **Test** | **Specification** | **Initial** | **9U Days** | **180 Days** |
|---|---|---|---|---|
| Aspect | Clear solution, transparent, free of impurities, with characteristic flavor and scent of Tutti-Frutti | Accordingly | Accordingly | Accordingly |
| pH | 6.0 - 7.0 | 6.5 | 6.4 | 6.5 |
| Clarity of Solution (Clearness) | Turbidity smaller than that of a kaolin suspension at 0.0005% in water | Accordingly | Accordingly | Accordingly |
| Desloratadine Content | 0.45 - 0.55mg/mL (90 - 110%) | 0.50 mg/mL (100%) | 0.48 mg/mL (96%) | 0.49 mg/mL (96%) |
| Prednisolone Content | 1.80 - 2.20 mg/mL (90 - 110%) | 2.01 mg/mL (101%) | 2.05 mg/mL (103%) | 2.00 mg/mL (100%) |
| Products of Degradation | Notification Limit: Max. 0.10% | Deg 1: 0.014% - ALN | Deg 1:0.00% - ALN | Deg 1:0.05% - ALN |
| | Identification Limit: Max. 0.20% | | | |
| | Qualification Limit: Max. 0.31% | | | |
| Total Aerobic Bacteria Count | Max. 100 UFC/mL | <10 UFC/ml | - | <10 UFC/mL |
| Molds / Yeasts Count | Max. 10 UFC/mL | <10 UFC/mL | - | <10 UFC/mL |
| Escherichia coli | Absent | Absent | - | Absent |
| Salmonella zo | Absent | Absent | - | Absent |
| | Absent | Absent | - | Absent |
| Pseudomonas aeroginosa | Absent | Absent | - | Absent |
| | | | | |
| Deg | Products of Degradation | | | |
| ALN | Smaller or equal to the Notification Limit | **Conclusion**: The results found on the stability Study allow the establishment of a period of validity of 24 months | | |

As shown in Table 1, the formulation of the invention possesses the desired characteristics to ensure the viability of the same for 24 months.

### Example 3: Super-Accelerated Stability Test of the Formulation of the Invention after 30 Days of Production

Concomitantly, the super-accelerated stability study was conducted (conditions: temperature 50° C and relative humidity of 90%; test duration time: 30 days) the formulation of the invention was packaged in amber bottles, as specified above.

The results are shown in Table 2.

**Table 2: Super-Accelerated Stability Study of the Formulation of Example 1**

| Test | Specification | Method | PD49 Initial | PD49 40°C/75%RH 40 Days | PD49 50°C/90%RH 30 Days |
|---|---|---|---|---|---|
| Aspect | Clear solution, colorless to slightly yellowish, with flavor and scent of Tutti-Frutti. Free of particles and strange material | Visual | Accordingly | Accordingly | Accordingly |
| pH | 6.0-7.0 | | 6.66 | 6.53 | 6.65 |
| Density | 1.070g/mL - 1.100g/mL | | 1.094 | 1.094 | 1.092 |
| Viscosity | 10 cP - 30 cP | | 17.00 | 17.10 | 17.10 |
| Desloratadine Content | 0.45 - 0.55 mg/mL (90 - 110%) | DMA12-023-00 | 0.52 mg/mL 104% | 0.50 mg/mL 100% | 0.48 mg/mL 96% |
| Prednisolone Content | 3.60-4.40 mg/mL (90 - 110%) | DMA12-023-00 | 4.27 mg/mL 107% | 4.06 mg/mL 102% | 3.74 mg/mL 94% |
| Products of Degradation (Desloratadine) | | DMA12-023-00 | Not detected | Not detected | |
| | | | | | RRT 0.93:0.94% - ALQ |
| | Notification Limit: Max. 0.10% | | | | RRT 1.35:0.34% - ALQ |
| | Identification Limit: Max. 0.20% | | | | |
| | Qualification Limit: Max. 0.31% | | | | |
| Products of Degradation (Prednisolone) | | DMA12-023-00 | Not detected | Not detected | |
| | | | | | RRT 0.49:0.11% - ALN |
| | | | | | RRT 031:0.04 % - ALN |

All publications and patent applications mentioned in this specification are indicative of the level of those skilled in the art to which the invention relates. All publications and patent applications are incorporated herein by reference to the same extent as if each individual publication or patent application were each specifically and individually indicated to be incorporated for ease of reference.

Although certain embodiments have been described, they are presented in an exemplary mode only, and are not intended to limit the scope of the invention. In fact, the new embodiments described herein may be implemented in a variety of other forms; more than that, various omissions, substitutions and changes in the form of the embodiments described herein may be made without departing from the spirit of the invention. The accompanying claims and their equivalents in this description are considered to cover such forms or modifications as they may be within the scope and spirit of the invention.

## Claims

1. Liquid pharmaceutical composition for oral administration comprising:
(i) a therapeutically effective quantity of desloratadine or a pharmaceutically acceptable salt thereof;
(ii) a therapeutically effective quantity of prednisolone or a pharmaceutically acceptable salt thereof; and
(iii) a pharmaceutically acceptable selection of excipients wherein the flavoring excipient used is free of vanillin or a derivative thereof, and wherein the pH of said composition is maintained at a value in the range between 6.0 and 7.5.

2. Composition according to claim 1, **characterized in that** the pH is maintained between 6.5 and 7.5.

3. Composition according to claim 2, **characterized in that** the pH is maintained at value of 7.0.

4. Composition according to claim 1, **characterized in that** said selection comprises pharmaceutically acceptable excipients: one or more surfactants; one or more thickeners; one or more flavoring agents free of vanillin or derivatives thereof; one or more sweetening agents; a suitable buffer system to maintain pH in the range 6.0 to 7.5; one or more stabilizing agent; and one or more solvents or carrier system.

5. Composition according to claim 4, **characterized in that** said one or more surfactants is selected from the group consisting of soybean lecithin, castor oil, corn oil, cottonseed oil, sunflower oil, peanut oil, polysorbate 20, polysorbate 60, polysorbate 80 and mixtures thereof.

6. Composition according to claim 5, **characterized in that** said surfactant is polysorbate 20 and is present in the composition at a concentration in the range of 0.1 to 0.5%.

7. Composition according to claim 4, **characterized in that** said one or more thickening agents is selected from the group consisting of hyetellose, guar gum, gelatin, xanthan gum, tamarind gum, tragacanth gum, karaya gum, povidone, sodium carboxymethylcellulose, sodium alginate, pectin, carrageenan, polyethylene glycol, modified starch, cellulose derivatives such a s c arboxymethylcellulose, hydroxymethylcellulose, hydroxypropylmethylcellulose, hydroxyethylcellulose, methylcellulose and microcrystalline cellulose.

8. Composition according to claim 7, **characterized in that** said thickening agent is hyetellose and is present in the composition at a concentration in the range of 0.1 to 2.0%.

9. Composition according to claim 4, **characterized in that** said one or more flavoring agents are free of vanillin or a derivative thereof, to be selected from the group consisting of derivatives of essential oils such as mint, lemon, orange, peppermint, eucalyptus; or based on organic synthetic blends like cherry, strawberry, pineapple, caramel, Tutti-Frutti, honey and fruit salad.

10. Composition according to claim 9, **characterized in that** said vanillin free flavoring agent or a derivative thereof is the essence of Tutti-Frutti or a derivative thereof, which has had the vanillin component removed, said Tutti-Frutti essence being present in the composition at a concentration in the range from 0.05 to 0.5%.

11. Composition according to claim 4, **characterized in that** said one or more sweetening agents is selected from the group consisting of sucralose, sodium saccharin, acesulfame potassium, sorbitol, xylitol, maltodextrin, fructose, stevia, aspartame, sodium cyclamate, glycyrrhizinate monoammonium.

12. Composition according to claim 11, **characterized in that** said one or more sweetening agents is the mixture of acesulfame potassium, sucralose and sodium saccharin, said mixture being present in the composition at a concentration in the range of 0.01 to 5%, 0%.

13. Composition according to claim 4, **characterized in that** said suitable buffer system to maintain pH in the range 6.0 to 7.5 is selected from the citrate buffers, phosphate, tartrate, fumarate, and maleic.

14. Composition according to claim 13, **characterized in that** said buffer system is the citrate buffer, in which citric acid and sodium citrate are in a ratio in the range 8:1 to 20:1.

15. Composition according to claim 14, **characterized in that** said buffer system is the citrate buffer, in which citric acid and sodium citrate are in the ratio of 17:1.

16. Composition according to claim 4, **characterized in that** said one or more stabilizing agents is EDTA or a salt thereof selected from dipotassium edetate, disodium edetate, sodium edetate and trisodium edetate, and being present in the composition at a concentration in the range of 0.005 to 0.1%.

17. Composition according to claim 4, **characterized in that** said one or more solvents or carrier system is selected from the group consisting of water, alcohols, glycols, propylene glycol, sorbitol, polyethylene glycol and glycerin.

18. Composition according to claims 4 and 17, **characterized in that** said one or more solvents or carrier system is the mixture of water, propylene glycol and sorbitol; propylene glycol being present in the composition at a concentration in the range of 10 to 30%; and sorbitol being present in the composition at a concentration in the range of 20 to 50%.

19. Composition according to claim 4, **characterized in that** said one or more preservatives is selected from the group consisting of benzoic acid, benzyl alcohol, methylparaben, ethylparaben, propylparaben, butylparaben and mixtures thereof.

20. Composition according to claim 19, **characterized in that** said one or more preservatives is present in the composition in a concentration ranging from 0.05% to 2%.

21. Composition according to claim 19 to 20, **characterized in that** said preservative is methylparaben in the concentration range of 0.05% to 0.5%.

22. Composition according to claim 21, **characterized in that** methylparaben is present in the composition at a concentration of 0.18%.

23. Liquid pharmaceutical composition for oral administration comprising:
(i) as active ingredients, desloratadine or a pharmaceutically acceptable salt thereof and prednisolone or its pharmaceutically acceptable salt; and
(ii) as the selection of excipients: the stabilizer EDTA; the mixture of sweeteners acesulfame potassium, sucralose and saccharin; citric acid buffer system and sodium citrate dihydrate at a ratio of 17:1; the thickener hyetellose; the preservative methylparaben; the surfactant polysorbate 20; the solvent system comprising water, propylene glycol and sorbitol; and the flavoring agent Tutti-Frutti that is free of vanillin or a derivative thereof,
wherein the pH of said composition is maintained at a value in the range between 6.0 and 7.5.

24. Liquid pharmaceutical composition for oral administration according to claim 23, **characterized in that** the pharmaceutically acceptable salt of prednisolone is prednisolone sodium phosphate.

25. Liquid pharmaceutical composition for oral administration according to any one of claims 1 to 24, **characterized in that** it is an antihistamine composition in the form of oral solution, intended for pediatric use.

26. Use of the pharmaceutical composition as according to any one of claims 1 to 25, for the preparation of a medication for the treatment of children suffering from allergic disorders or inflammation resulting from said allergic manifestations.

27. Use of the pharmaceutical composition according to claim 23, **characterized in that** the allergic manifestation is allergic rhinitis.

28. Use of the pharmaceutical composition according to claim 23, **characterized in that** it is in the form of an oral solution.
